# EUROPEAN PATENT APPLICATION

(11) **EP 1 749 476 A1**
(43) Date of publication of application: **07.02.2007**
(21) Application number: 05743864.0
(22) Date of filing: 25.05.2005
(51) Int. Cl.: A61B 5/145, A61B 5/00, G01N 21/27

(54) **OPTICAL ELEMENT FOR MEASURING BIOINFORMATION AND BIOINFORMATION MEASURING DEVICE**

(30) Priority: 26.05.2004 JP 2004156049
(71) Applicant: MATSUSHITA ELECTRIC INDUSTRIAL CO., LTD., Kadoma-shi, Osaka 571-8501 (JP)
(72) Inventor: SHIOI, Masahiko Matsushita Electric Ind. Co., Ltd., Chuo-ku, Osaka-shi, Osaka 540-6319 (JP); UCHIDA Matsushita Electric Ind. Co., Ltd., Chuo-ku, Osaka-shi, Osaka 540-6319 (JP); OSHIMA, Kiyoko Matsushita Electric Ind. Co., Ltd., Chuo-ku, Osaka-shi, Osaka 540-6319 (JP)
(74) Representative: TBK-Patent
(86) International application number: PCT/JP2005/009561
(87) International publication number: WO 2005/115244

(57) **Abstract**

To achieve a reliable and easy measurement of a concentration of a target component in an organism, in an optical element including: a first light-entrance plane, a body tissue contact-plane provided with a groove portion to be brought into contact with a body tissue, and a first light-exit plane; the groove portion includes a second light-entrance plane and a second light-exit plane; light that entered through the first light-entrance plane is applied to the body tissue protruded by the groove portion via the second light-entrance plane, and light absorbed by the body tissue and/or scattered, and entered through the second light-entrance plane exits the first light-exit plane; and a normal line of the plane opposing the body tissue contact-plane, and a plane made by a normal line of the second light-entrance plane and a normal line of the second light-exit plane intersect with each other.

## Description

### TECHNICAL FIELD

The present invention relates to an optical element used for optically measuring a body tissue to measure glucose, cholesterol, urea, and triglycerides in body fluids noninvasively, and to a biological information measuring device using the optical element.

### BACKGROUND ART

Conventionally, various optical measuring methods have been proposed for measuring a specific component in an organism and a solution by using an optical measuring device. For example, Patent Document 1 proposed a contact element for detecting biological information, comprising an abutting means having a recess portion to be abutted to a body tissue; a detecting-light outputting means for outputting detecting-light from a portion in the recess portion; and a detecting-light introducing means provided at a portion in the recess portion for introducing the detecting-light, wherein said abutting means is formed of a material having a higher refractive index than the refractive index of the body tissue.

In the above contact element, the detecting-light passes through the body tissue that entered into the recess portion while the abutting means and the body tissue are in contact, and then enters the detecting-light introducing means. Such contact element achieves easier handling, less damage to the body tissue, and easy and highly accurate measurement of biological information.
Patent Document 1 : International Publication No. 01/058355

### DISCLOSURE OF THE INVENTION

### PROBLEM TO BE SOLVED BY THE INVENTION

However, conventional optical measurement method and optical measuring device using such a contact element as in the above had following problems.
In the technique described in Patent Document 1, the body tissue is made to protrude so that the tissue goes into the formed recess portion, and the measurement can be conducted selectively for the protruded portion. However, when the light enters into the body tissue from the recess portion, at the interface between the recess portion and the body tissue, the incident light is refracted depending upon the refractive indexes of the contact element for detecting biological information and the body tissue, and enters into the body tissue. At this time, simultaneously, reflected light is generated also at the interface between the body tissue and the recess portion. This reflected light keeps reflecting in the contact element for detecting biological information, at the interface between the contact element and the outside contact element.

Some of such multiple reflected light form an evanescent wave due to total reflection between the contact element and the outside contact element, and are absorbed by a portion of the body tissue where the measurements are not preferable and by those existing outside the contact element.
Some of the multiple reflected light enter the light-detector after repeating the reflection. The reflected light thus entered the light-detector overlaps with the target light to be detected, causing an error and making the stable measurement of biological information difficult.

The present invention is made in view of the above situations, and aims to provide an optical element for measuring biological information that enables reliable and easy measurement of body fluid components in a sample noninvasively and a biological information measuring device using the optical element.

### MEANS FOR SOLVING THE PROBLEM

To solve the above conventional problems, the present invention provides an optical element for measuring biological information comprising:
a first light-entrance plane, a body tissue contact-plane provided with a groove portion to be brought into contact with a body tissue, and a first light-exit plane,
   the groove portion comprising a second light-entrance plane and a second light-exit plane,
wherein light entering through the first light-entrance plane is applied to a body tissue protruded by the groove portion via the second light-exit plane, and light absorbed by the body tissue and/or be scattered, and entering through the second light-entrance plane exits from the first light-exit plane; and
a normal line of the plane opposing the body tissue contact-plane, and a plane made by a normal line of the second light-entrance plane and a normal line of the second light-exit plane intersect with each other.

The present invention also provides a biological information measuring device comprising:
a light source,
the above optical element,
a light-detector for detecting light exiting said optical element, and
a computing unit for computing information obtained by the light-detector.

### EFFECT OF THE INVENTION

Based on the present invention, in a biological information measuring device comprising an optical element provided with a groove portion, the effects of the reflected light generated in the optical element are mitigated, and a concentration of the target component in a body tissue can be measured with reliability and ease.

### BRIEF DESCRIPTION OF THE DRAWINGS

[FIG. 1] A perspective view schematically illustrating a form of embodiment 1 of an optical element for measuring biological information in the present invention.
[FIG. 2] A cross-sectional view taken along the line X-X in FIG. 1.
[FIG. 3] An enlarged view of a groove portion 12a in FIG. 2.
[FIG. 4] A cross-sectional view taken along the line Y-Y in FIG. 1.
[FIG. 5] A perspective view schematically illustrating an example of a variation of an optical element in embodiment 1 of the present invention.
[FIG. 6] A cross-sectional view taken along the line Y-Y in FIG. 5.
[FIG. 7] A perspective view schematically illustrating an optical element for measuring biological information in embodiment 2 of the present invention.
[FIG. 8] A cross-sectional view taken along the line Y-Y in FIG. 7.
[FIG. 9] A perspective view schematically illustrating an optical element for measuring biological information in embodiment 3 of the present invention.
[FIG. 10] A cross-sectional view taken along the line Y-Y in FIG. 9.
[FIG. 11] A perspective view schematically illustrating an optical element for measuring biological information in embodiment 4 of the present invention.
[FIG. 12] A perspective view schematically illustrating a variation of an optical element in embodiment 4 of the present invention.
[FIG. 13] A schematic view of a biological information measuring device in an embodiment of the present invention.

### BEST MODE FOR CARRYING OUT THE INVENTION

An optical element for measuring biological information of the present invention comprises a first light-entrance plane, a body tissue contact-plane provided with a groove portion to be brought into contact with a body tissue, and a first light-exit plane,
the groove portion comprising a second light-entrance plane and a second light-exit plane,
wherein light entering through the first light-entrance plane is applied to the body tissue protruded by the groove portion via the second light-exit plane, and light absorbed by the body tissue and/or scattered, and entering through the second light-entrance plane exits from the first light-exit plane; and
a normal line of a plane opposing the body tissue contact-plane, and a plane made by a normal line of the second light-entrance plane and a normal line of the second light-exit plane intersect with each other.
Based on such a structure, reflected light generated at the groove portion can be prevented from entering the light-detector, and a concentration of a target component in a test sample can be measured reliably and easily.

In the case when there are a plurality of the normal lines of the plane opposing the body tissue contact-plane (for example, in the case when there are a plurality of the planes opposing the body tissue contact-plane, and in the case when the plane opposing the body tissue contact-plane is a curved plane), the effects of the present invention as described above can be achieved when at least one of the plurality of normal lines and the plane made by the normal line of the second light-entrance plane and the normal line of the second light-exit plane intersect with each other.

In the optical element for measuring biological information of the present invention, the plane opposing the body tissue contact-plane preferably comprises a recess portion.
A cross section of the recess portion may be substantially V-shaped, and the recess portion may be formed with a curved plane.
Also, in the optical element for measuring biological information of the present invention, the plane opposing the body tissue contact-plane may comprise a projecting portion.
The projecting portion is preferably formed with a curved plane.
In the optical element for measuring biological information of the present invention, the body tissue contact-plane preferably comprises a flow path with an opening portion.
A bottom plane of the flow path may be substantially V-shaped, and may be a curved plane.

Additionally, a biological information measuring device of the present invention comprises a light source, the above optical element, a light-detector for detecting light exiting the optical element, and a computing unit for computing information obtained by the light-detector.
Since the biological information measuring device of the present invention uses the optical element of the present invention as mentioned, the effects from reflected light generated at the groove portion of the above optical element can be mitigated, and a concentration of a target component in a test sample can be measured reliably and easily.

In the following, embodiments of the present invention are described in detail by referring to the drawings, but the present invention is not limited to these embodiments. Also, in the description below, same reference numerals are used for the same or corresponding part, and repetitive descriptions may be omitted.

### [Embodiment 1]

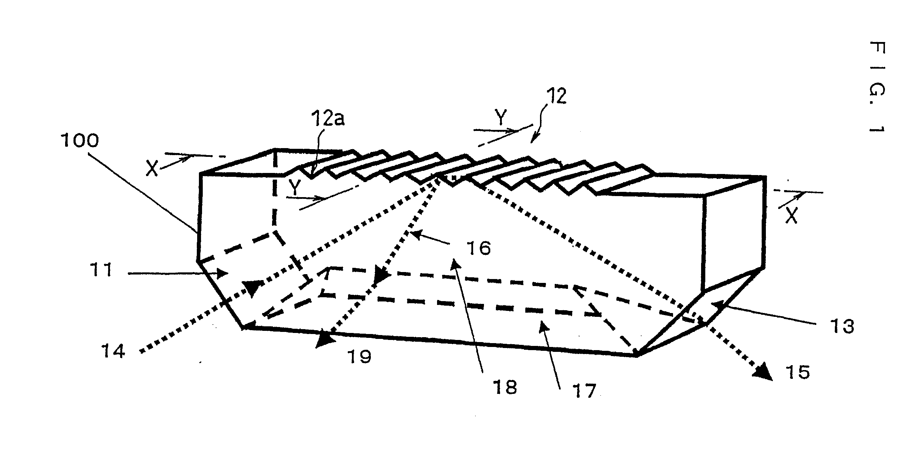

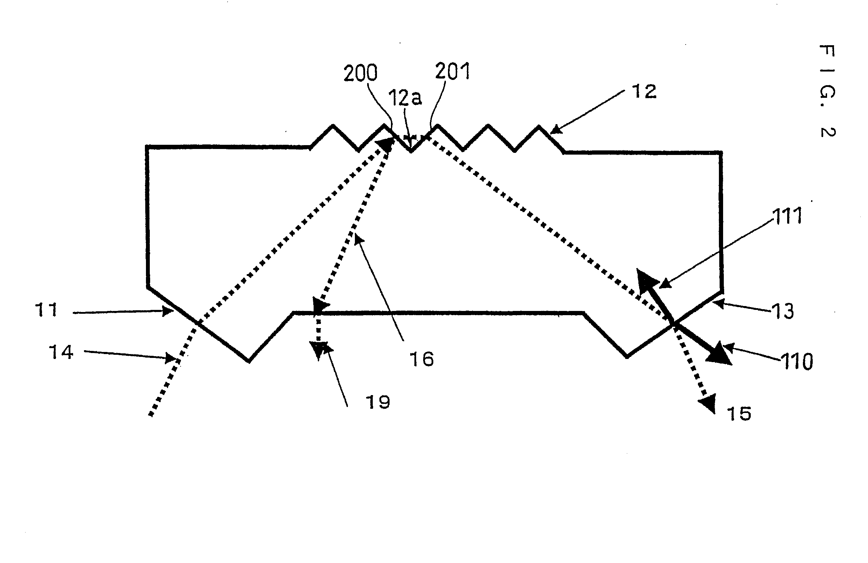

FIG. 1 is a perspective view schematically illustrating an optical element for measuring biological information in embodiment 1 of the present invention. FIG. 2 is a cross-sectional view taken along the line X-X of the optical element for measuring biological information illustrated in FIG. 1.
As shown in FIGs. 1 and 2, an optical element 100 for measuring biological information in this embodiment comprises a first light-entrance plane 11, a body tissue contact-plane 12, and a first light-exit plane 13: the body tissue contact-plane 12 comprises a groove portion 12a to protrude a body tissue. The groove portion 12a comprises a second light-exit plane 200 and a second light-entrance plane 201. Further, at the plane opposing the body tissue contact-plane 12 (that is, a lower side of the optical element 100), a V-shaped recess portion having slope planes 17 is provided.

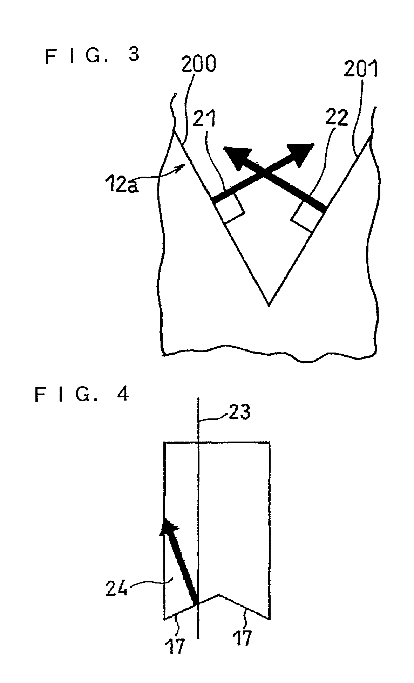

FIG. 3 is an enlarged view of the groove portion 12a in FIG. 2, and FIG. 4 is a cross-sectional view taken along the line Y-Y in FIG. 1. As shown in FIG. 3, the groove portion 12a has two planes, i.e., a light-entrance plane and a light-exit plane which have a normal line 21 and a normal line 22, respectively.
These two normal lines 21 and 22 form a plane 23, as shown in FIG. 4. When the normal line 21 and the normal line 22 are skew lines, starting points of respective normal lines 21 and 22 can be aligned to assume a plane.

At this time, a normal line 24 of the plane opposing the body tissue contact-plane (that is, the slope plane 17 provided at the V-shaped recess portion of a lower side of the optical element 100) and the plane 23 formed by the normal lines 21 and 22 of the groove portion 12a intersect with each other.
Although the V-shaped recess portion comprises two planes (slope planes 17), in FIG. 4, a normal line of one plane only is illustrated. The both normal lines of two planes of the V-shaped recess portion, and the plane 23 mentioned in the above preferably intersect with each other.

Functions of an optical element 100 for measuring biological information of the present invention are described next.
Light enters into the optical element 100 for measuring biological information through a first light-entrance plane 11. Incident light 14 that entered into the optical element 100 for measuring biological information is refracted at the interface between the groove portion 12a of the body tissue contact-plane 12 and the body tissue (not shown). The refracted light enters the body tissue through a second light-exit plane 200, and the light that entered into the body tissue is absorbed by the body tissue, refracted again at the body tissue and the groove portion 12a, and enters the optical element 100 for measuring biological information through a second light-entrance plane 201. The light that made a re-entrance into the optical element 100 for measuring biological information exits the optical element 100 for measuring biological information from the first light-exit plane 13 as outgoing light 15.

Reflected light is generated when light is refracted at the interface between the groove portion 12a and the body tissue. In the optical element 100 for measuring biological information of the present invention, the outgoing light 15 is used for measuring the body tissue, and reflected light focused in the present invention is reflected light 16 that is generated at the interface between the groove portion 12a and the body tissue.
In a usual optical element, the reflected light 16 is possibly reflected again to enter the body tissue, affecting the outgoing light 15 and causing an error in measurement results, whereas in the present invention, due to the presence of the slope plane 17, the reflected light 16 can be released to the outside as side-plane outgoing light 19 without entering into the body tissue again.

The reflected light 16 exits the optical element 100 for measuring biological information from a side-plane 18 adjoining the body tissue contact-plane 12 as the side-plane outgoing light 19, due to the slope plane 17 forming a V-shaped recess portion provided at a plane opposing the body tissue contact-plane 12.
The side-plane outgoing light 19 thus released does not reach a light-detector (not shown) provided ahead of the first light-exit plane 13, unlike the outgoing light 15 exited from the first light-exit plane 13.

Referring to FIG. 2, detailed description on functions of the slope planes 17 forming the V-shaped recess portion and a direction of the reflected light 16 is given next. However, in FIG. 2, less number of the grooves is illustrated in the groove portion 12a for simplification.
In a plane formed by a wave vector 110 of light reentered the optical element 100 for measuring biological information through the interface between the groove portion 12a provided at the body tissue contact-plane 12 and the body tissue and traveled toward the first light-exit plane 13, and a normal line vector 111 of the first light-exit plane 13, the light that entered through the first light-entrance plane 11 exists invariably. Therefore, usually, the reflected light 16 reflected at the interface between the groove portion 12a and the body tissue also invariably exists in the plane formed by the wave vector 110 and the normal line vector 111 of the first light-exit plane 13.

In the optical element 100 for measuring biological information, this reflected light 16 repeats reflection, or total reflection depending on conditions, and eventually exits from the first light-exit plane 13: the reflected light 16 thus detected by a light-detector provided ahead of the first light-exit plane 13 causes an error.
As opposed, in the optical element 100 for measuring biological information of the present invention, as mentioned in the above, by forming the V-shaped recess portion with the slope planes 17, the reflected light 16 is reflected at the slope planes 17, and eventually exits the optical element 100 for measuring biological information from the side-plane 18 as the side-plane outgoing light 19.

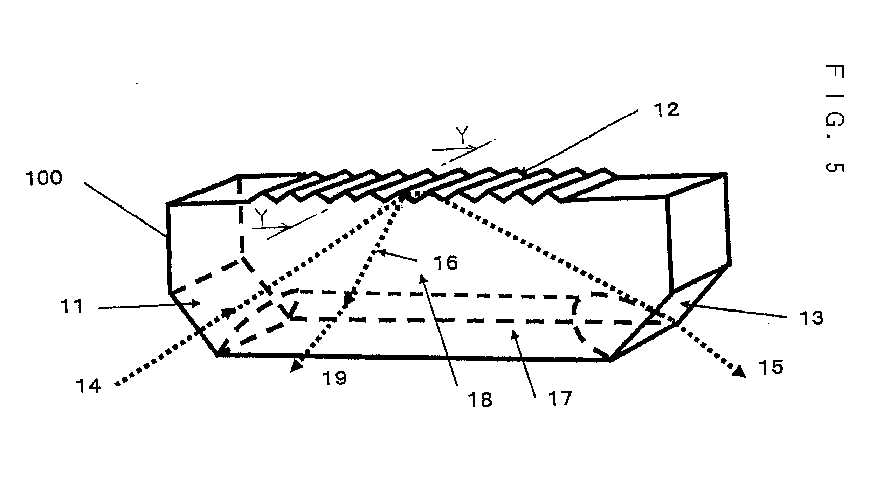

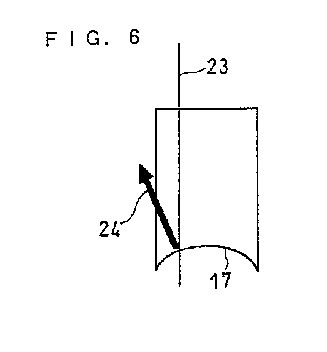

Although substantially V-shape was given to the recess portion at the lower side of the optical element 100 in the above embodiment 1, as shown in FIG. 5, the recess portion may be formed to give a substantially semicircular shape. FIG. 5 is a perspective view schematically illustrating a variation of an optical element in embodiment 1 of the present invention. FIG. 6 is a cross-sectional view taken along the line Y-Y in FIG. 5.
As shown in FIG. 6, in this case as well, a normal line 24 of a plane opposing the body tissue contact-plane (that is, a curved plane 17 provided at the semicircular recess portion at the lower side of the optical element 100) and the plane 23 formed by the normal lines 21 and 22 of the groove portion 12a intersect with each other.

### [Embodiment 2]

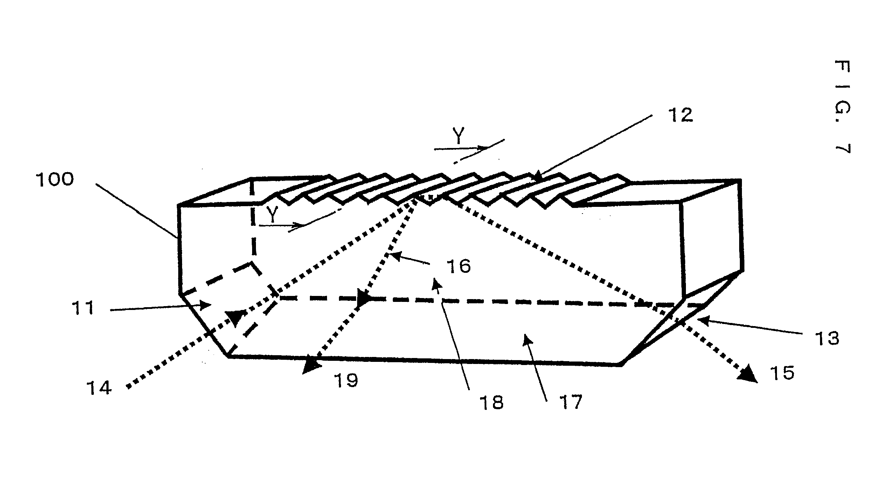

FIG. 7 is a perspective view schematically illustrating an optical element for measuring biological information in embodiment 2 of the present invention. In an optical element 100 for measuring biological information shown in FIG. 7, unlike FIGs. 1 and 4, a single slope plane 17 is provided, and the lower side as a whole is oblique to the body tissue contact-plane 12.
Based on such a structure, the same effects with the case of V-shaped recess portion shown in FIG. 1 are achieved, and in view of manufacturing process, the slope plane 17 can be processed easier compared with the case in embodiment 1.

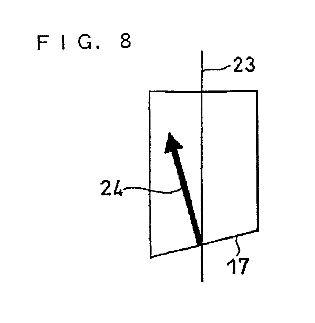

FIG. 8 is a cross-sectional view taken along the line Y-Y in FIG. 7. In the case of the optical element 100 of this embodiment as well, as in the embodiment 1 shown in FIG. 3, the groove portion 12a comprises two planes, i.e., a second light-entrance plane and a second light-exit plane which have a normal line 21 and a normal line 22, respectively. Then, these two normal lines 21 and 22 form a plane 23, as shown in FIG. 8.
A normal line 24 of the plane opposing the body tissue contact-plane (that is, a slope plane 17 at a lower side of the optical element 100) and a plane 23 formed by the normal lines 21 and 22 of the groove portion 12a intersect with each other.

### [Embodiment 3]

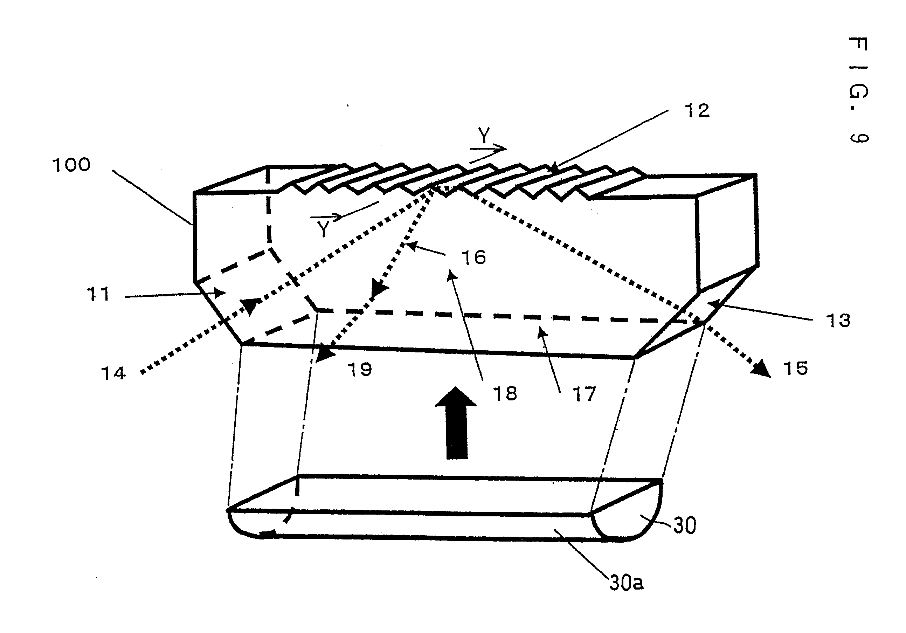

FIG. 9 is a perspective view schematically illustrating an optical element for measuring biological information in embodiment 3 of the present invention. In the optical element 100 of the present invention, the plane opposing the body tissue contact-plane 12 may be a curved plane.
In such a case, as shown in FIG. 9, by attaching a semicylindrical member 30 at a planar lower side portion opposing the body tissue contact-plane 12 (lower plane 17), a curved plane 30a can be formed. The curved plane 30a can be formed by machining and a removal processing method such as etching.

The removal processing method such as cutting or etching techniques may be used to form the V-shaped recess portion having the slope planes 17 in FIG. 1, the curved plane recess portion in FIG. 5, and the single slope plane 17 in FIG. 7.
Additionally, for example, the V-shaped recess portion may also be formed by attaching two triangular prism members at the planar lower plane.

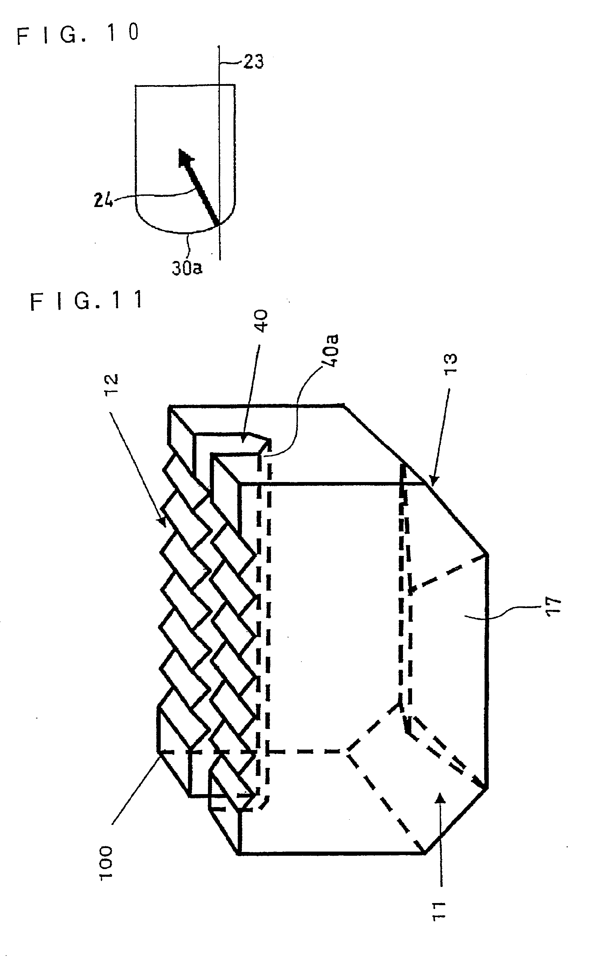

FIG. 10 is a cross-sectional view taken along the line Y-Y in FIG. 9. In the optical element 100 of this embodiment as well, as in embodiment 1 illustrated in FIG. 3, the groove portion 12a comprises two planes, i.e., a second light-entrance plane and a second light-exit plane which have normal lines 21 and 22, respectively. These two normal lines 21 and 22 form a plane 23, as shown in FIG. 10.
At this time, a normal line 24 of the plane opposing the body tissue contact-plane (that is, a curved plane 30a at the lower side of the optical element 100) and the plane 23 formed by the normal lines 21 and 22 of the groove portion 12a intersect with each other.

### [Embodiment 4]

FIG. 11 is a perspective view schematically illustrating an optical element for measuring biological information in embodiment 4 of the present invention. In the optical element 100 of the present invention, the body tissue contact-plane 12 may be provided with a flow path 40, i.e., the body tissue contact-plane 12 having a opening portion.
This flow path 40 is, for example, for liquids such as saliva, sweat, water to pass through. Such flow path 40 is useful in that sweat, saliva, and the like secreted from the body tissue, and water are removed from the body tissue contact-plane 12 to prevent an error in measurement.

When the bottom plane of flow path 40 is planar, reflected light reflected at the bottom plane of the flow path 40 usually does not enter the light-detector provided ahead of the first light-exit plane 13 as described in the above, due to the presence of the slope plane 17 provided at the plane opposing the body tissue contact-plane 12.
However, some reflected light may reflect at the bottom of the flow path 40 and reach directly to the first light-exit plane 13, and may be detected by the light-detector. Since the flow path 40 is filled with those substances not including biological information such as sweat and saliva, the light reflected at the bottom plane of the flow path 40 causes an error when detected by the light-detector.

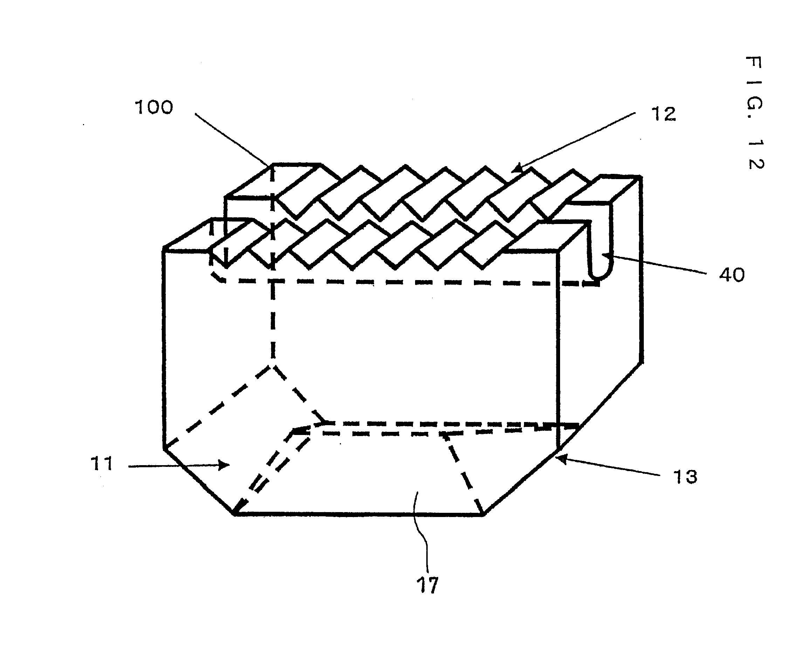

Thus, by providing a slope plane 40a at the bottom plane of the flow path 40 as shown in FIG. 11, based on the same principle with the case described with reference to FIG. 1, reflected light is allowed to exit from the side-plane adjoining the body tissue contact-plane 12 and removed, so that the reflected light does not affect the measurement. Further, at the time of processing the flow path 40, by using a V-shaped cutting tool, V-shaped bottom plane with a slope plane is given to the flow path, which makes the processing easy: therefore, the bottom plane of the flow path 40 is preferably V-shaped.
Although the slope plane is provided at the bottom plane of the flow path 40, as shown in FIG. 12, the bottom plane of the flow path 40 may be a curved plane. FIG. 12 is a perspective view schematically illustrating a variation of an optical element in embodiment 4 of the present invention.

### [embodiment 5]

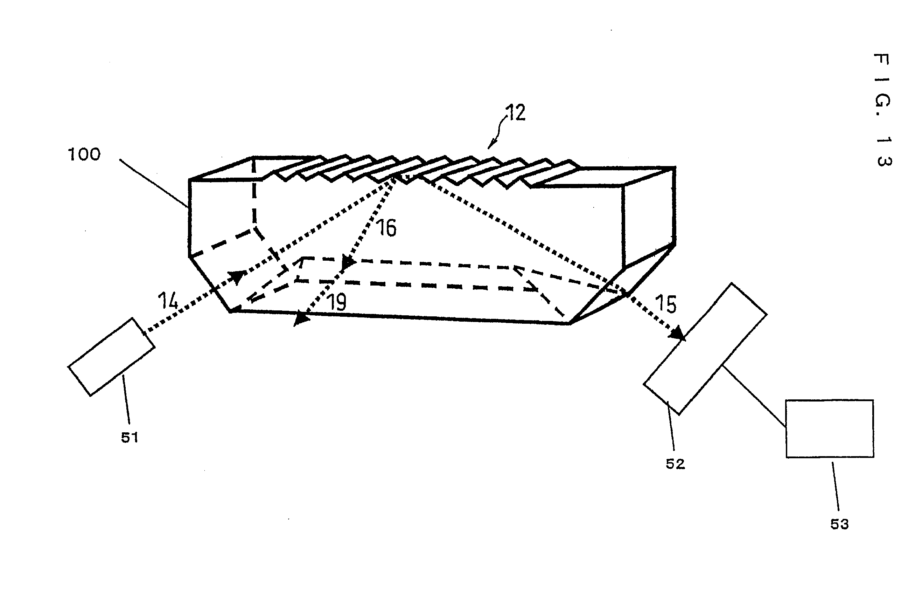

FIG. 13 is a schematic illustration of a form of a biological information measuring device in one embodiment of the present invention. As shown in FIG. 13, the biological information measuring device in this embodiment is formed with a light source 51, an optical element 100 for measuring biological information, a light-detector 52, a computing unit 53, and a spectroscope (not shown) provided between the light source 51 and the optical element 100 for measuring biological information. For the spectroscope, for example, a spectroscope using grating, and Fourier transform spectroscope may be used.

Operation of the biological information measuring device is described next by referring to FIG. 13.
Light emitted from the light source 51 enters the optical element 100 for measuring biological information. Incident light 14 that entered into the optical element 100 for measuring biological information passes through the body tissue (not shown) contacting the body tissue contact-plane 12 and scatters, and exits the optical element 100 for measuring biological information. Outgoing light 15 exited the optical element 100 for measuring biological information is detected by the light-detector 52, and biological information is calculated by the computing unit 53.

For the light source 51, the one that generates light having an absorption wavelength of the component to be measured, i.e., the measurement target, in the body tissue will suffice. For example, a light-emitting diode (LED), a halogen light source, a semiconductor laser, a globar light source which is SiC sintered into a rod, a CO₂ laser, and a tungsten lamp may be mentioned.

When a substance having an absorption peak in the mid-infrared region such as wavenumbers of 1033cm⁻¹ and 1080cm⁻¹, and in the near-infrared region of 1 to 2.5 µm, such as glucose, is to be measured, the following light sources are preferably used. That is, when the measurement is to be carried out with light in the mid-infrared region, a globar light source is preferable in view of a comparatively wide wavelength range coverage, and excellent radiation can be achieved also in the long wavelength range of about 10 µm. In the case when light in the near-infrared region is to be measured, a halogen light source is preferable. Regardless to say, since glucose has an absorption peak also in the near-infrared region, a light source other than the above may also be used.

For the material for the optical element 100 for measuring biological information, known ones in the art can be used based on the wavelength of the light used for the measurement. For example, silicon, germanium, SiC, diamond, ZnSe, ZnS, fused quartz, calcium fluoride, and KrS may be mentioned.

When a substance having an absorption peak in the mid-infrared region such as wavenumbers of 1033 cm⁻¹ and 1080cm⁻¹ and in the near-infrared region of 1 to 2.5 µm, such as glucose, is to be measured, the following materials are preferably used. That is, in view of the fact that in the mid-infrared region, the transmittance is high in the infrared wavelength of about 9 to 10 µm, silicon or germanium with a small content of impurity such as boron and phosphorus, and a resistivity of 100 Ωcm or more is preferable, and a resistivity of 1500 Ωcm or more is further preferable.
On the other hand, when a substance having an absorption peak in the near-infrared region of 1 to 2.5 µm is to be measured, silicon with a resistivity of 100 Ωcm or more is preferable, and a resistivity of 1500 Ωcm or more is further preferable, and calcium fluoride, fused quartz may be used as well.

For the light-detector 52, known ones in the art can be used without particular limitation, for example, in the case of the mid-infrared region, an MCT detector (mixed crystal of mercury, tellurium, and cadmium), a pyroelectric sensor, a DTGS detector, a thermistor, a thermopile, Golay cell may be mentioned. Also, in the case of the near-infrared region, a PbS detector, an InSb detector, a PbSe detector, and an InGaAs detector may be mentioned.
For the computing unit 53, a computer is preferably used.

### INDUSTRIAL APPLICABILITY

As described in the above, the optical element for measuring biological information of the present invention and a biological information measuring device using the optical element cause less measurement error from reflected light, and are useful for measurement of body fluid components, particularly for medical purposes.

## Claims

1. An optical element for measuring biological information, comprising:
a first light-entrance plane,
a body tissue contact-plane provided with a groove portion to be brought into contact with a body tissue, and
a first light-exit plane,
said groove portion comprising a second light-entrance plane and a second light-exit plane,
wherein light entering through said first light-entrance plane is applied to the body tissue protruded by said groove portion via said second light-entrance plane, and light absorbed by said body tissue and/or scattered, and entering through said second light-entrance plane exits from said first light-exit plane; and
a normal line of a plane opposing said body tissue contact-plane, and a plane made by a normal line of said second light-entrance plane and a normal line of said second light-exit plane intersect with each other.

2. The optical element for measuring biological information in accordance with claim 1, wherein said plane opposing said body tissue contact-plane comprises a recess portion.

3. The optical element for measuring biological information in accordance with claim 2, wherein a cross section of said recess portion is substantially V-shaped.

4. The optical element for measuring biological information in accordance with claim 2, wherein said recess portion is formed with a curved plane.

5. The optical element for measuring biological information in accordance with claim 1, wherein said plane opposing the body tissue contact-plane comprises a projecting portion.

6. The optical element for measuring biological information in accordance with claim 5, wherein said projecting portion is formed with a curved plane.

7. The optical element for measuring biological information in accordance with any of claims 1 to 6, wherein said body tissue contact-plane comprises a flow path with an opening portion.

8. The optical element for measuring biological information in accordance with claim 7, wherein a bottom plane of said flow path is substantially V-shaped.

9. The optical element for measuring biological information in accordance with claim 7, wherein said bottom plane of the flow path is a curved plane.

10. A biological information measuring device comprising:
a light source,
the optical element in accordance with any of claims 1 to 9,
a light-detector for detecting light exiting said optical element, and
a computing unit for computing information obtained by said light-detector.
